# FASCICULE DE BREVET EUROPEEN

(11) **EP 0 369 887 B1**
(45) Date de publication et mention de la délivrance du brevet: **21.06.1995**
(21) Numéro de dépôt: 89403164.0
(22) Date de dépôt: 17.11.1989
(51) Int. Cl.: A61K 31/44

(54) **Utilisation de trifluorométhylphényltétrahydropyridines pour la préparation de médicaments destinés à combattre les troubles anxio-dépressifs**
Verwendung von Trifluormethylphenyl-Tetrahydropyridinen zur Herstellung von Arzneimitteln zur Behandlung anxio-depressiver Erkrankungen
Use of trifluoromethyl phenyl-tetrahydropyridines in the manufacture of a medicament for the treatment of anxio-depressive disorders

(30) Priorité: 18.11.1988 FR 8815036
(43) Date de publication de la demande: 23.05.1990
(73) Titulaire: SANOFI, 75008 Paris (FR)
(72) Inventeur: Bianchetti, Alberto, I-20122 Milan (IT); Le Fur, Gérard, F-95160 Montmorency (FR); Simiand, Jacques, F-31600 Muret (FR); Soubrie, Philippe, F-34270 St Mathieu de Treviers (FR)
(74) Mandataire: Gillard, Marie-Louise

(56) Documents cités:
- EP-A- 0 060 179
- EP-A- 0 101 381
- EP-A- 0 164 633
- EP-A- 0 253 146
- US-A- 4 485 109
- THE JOURNAL OF PHARMACOLOGY AND EXPERIMENTAL THERAPEUTICS, vol. 248, no. 3, mars 1989, pages 1222-1230, The American Society for Pharmacology and Experimental Therapeutics, US; J. ADRIEN et al.: "Biochemical and electrophysiological evidence for an agonist action of CM 57493 at pre- and postsynaptic 5-hydroxytryptamine1A receptors in brain"
- TRENDS PHARMACOL. SCI, vol. 8, 1987, pages 432-437, Elsevier Publications, Cambridge, GB; J. TRABER et al.: "5-HT1A receptor-related anxiolytics"
- Textbook of Pharmacology - 2nd ed. - W.C. Bouwman, M.I. Rand, 15.5 Burger's Medicinal Chemistry, 4th ed., Part. III, J.Willy & Sons N.Y., 939-41

## Description

La présente invention concerne l'utilisation de trifluorométhylphényltétrahydropyridines pour la préparation de médicaments destinés à combattre l'anxiété et en général les troubles anxio-dépressifs.

Les brevets européens EP 60176 et EP 101381 décrivent des trifluorométhylphényltétrahydropyridines N-substituées à action anorexigène.

La demande de brevet EP 164 633 décrit des dérivés d'indoles ayant une activité sur le système nerveux central.

Le brevet US 4 485 109 décrit des 4-aryl-4-pipéridinecarbinols utiles comme anti-dépresseurs et dans certains cas comme agents anorexigènes.

On a maintenant trouvé que lesdites trifluorométhylphényltétrahydropyridines possèdent une bonne activité anxiolytique et antidépressive.

On a également trouvé que l'activité anxiolytique des mêmes composés n'est pas accompagnée, aux doses anxiolytiques, d'un effet sédatif.

On a enfin trouvé que les actions anxiolytique et antidépressive se manifestent à des doses nettement inférieures à celles qui donnent l'anorexie.

Ainsi, la présente invention concerne l'utilisation de trifluorométhylphényltétrahydropyridines de formule : 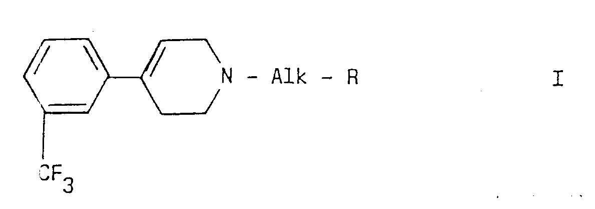
dans laquelle Alk représente un groupe alkylène à chaîne droite ou ramifiée ayant de 1 à 4 atomes de carbone et R représente un groupe cyano, un groupe acétyle, un groupe cycloalkyle ayant 3 à 7 atomes de carbone, un groupe pyridyle, un groupe pyridyle 1-oxyde ou un groupe naphtyle, et de leurs sels pharmaceutiquement acceptables, comme principes actifs pour la préparation de médicaments destinés à combattre l'anxiété et les troubles anxio-dépressifs.

Les composés de formule I et leur préparation sont décrits dans EP 60176 et EP 101381.

Lorsque, dans la formule I, R représente le groupe pyridyle, ce groupe est attaché à Alk par un des atomes de carbone en position 2, 3 ou 4; lorsque R représente le groupe naphtyle, il est attaché à Alk soit en position 1, soit en position 2.

Les composés de formule I dans laquelle Alk est le groupe éthylène et R est un groupe 2-pyridyle, 4-pyridyle, 1-oxyde-4-pyridyle, cyclohexyle ou 2-naphtyle et ceux de formule I dans laquelle Alk est un groupe éthylène, propylène ou butylène et R est le groupe cyano ainsi que leurs sels pharmaceutiquement acceptables, sont les plus intéressants, le 1-[ 2-(2-naphtyl)éthyl]-4-(3-trifluorométhylphényl)-1,2,3,6-tétrahydropyridine et ses sels pharmaceutiquement acceptables, notamment le chlorhydrate (composé de l'exemple 7 de EP 101381, ci-après désigné SR 57746 A), étant particulièrement avantageux.

Parmi les produits ci-dessus, le chlorhydrate de 4-(3-trifluorométhylphényl)-1-(2-cyanoéthyl)-1,2,3,6-tétrahydro_ pyridine (formule I, Alk = éthylène et R = cyano, CM 57493, décrit dans les exemples 2 et 15 de EP 60176) a été soumis à tous les tests nécessaires pour le passage en clinique humaine. Il s'est révélé très bien toléré et très peu toxique, sans signes particuliers d'activités secondaires gênantes. Testé chez l'homme à une dose de 80 et de 160 mg, le produit a été bien toléré et a montré un effet favorable sur l'humeur.

Les composés de formule I ont été essayés dans les tests prédictifs pour une activité anxiolytique et/ou antidépressive et se sont montrés actifs à très faibles doses, en justifiant ainsi leur utilisation comme anxiolytiques et/ou antidépresseurs pour la préparation de médicaments destinés à combattre l'anxiété et les troubles anxio-dépressifs.

L'administration des composés de formule (I) peut être effectuée par voie orale, sublinguale, transdermique ou par voie parentérale. La quantité de principe actif à administrer pour traiter les états anxio-dépressifs dépend, comme il est habituel, de la nature et de la sévérité des affections à traiter ainsi que du poids des malades.

La posologie chez l'être humain est variable entre 0,2 et 150 mg de produit par jour, administré en une à trois fois, les doses inférieures étant réservées aux enfants.

Pour la préparation de compositions pharmaceutiques selon la présente invention, les principes actifs (composés de formule I et leurs sels pharmaceutiquement acceptables) sont formulés en unités de dosage contenant de 0,2 à 150 mg, de préférence de 0,5 à 50 mg, dudit principe actif, seul ou en mélange avec un excipient pharmaceutique.

A titre d'exemple, lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent conterir l'ingrédient actif en mélange avec des agents de dispersion ou des agents mouillants, ou des agents de mise en suspension, tels que la polyvinylpyrrolidone et similaires, de même qu' avec des édulcorants ou des correcteurs du goût.

Le principe actif peut être formulé également sous forme de microcapsules, éventuellement avec un ou plusieurs supports ou additifs.

Pour une administration sublinguale, on peut préparer des micropastilles ou des microcapsules qui, lorsqu'ils sont placés sous la langue, se dissolvent très rapidement. Ces compositions en général contiennent l'ingrédient actif en mélange avec des agents mouillants et/ou des agents de dispersion et éventuellement avec des produits auxiliaires accélérant l'absorption.

Pour une administration transdermique, on peut envisager l'utilisation de matrices de diffusion polymériques pour la libération continue, de préférence sur une période de temps prolongée, du principe actif et l'utilisation du principe actif sous forme de microémulsions formées d'excipients compatibles avec la peau.

Pour une administration parentérale, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Les compositions pharmaceutiques sont préparées selon les méthodes connues, déjà décrites dans EP 60176 et EP 101381.

Les exemples suivants illustrent l'invention.

### Exemple 1

Un principe actif représentatif de la présente invention, le SR 57746 A, soumis au test de l'agressivité territoriale (A.T.) chez la souris selon C.Y. Yen et al. (Arch. Int. Pharmacodyn., 1959, 123,179-185), s'est montré actif à la dose orale de 1 mg/kg.

Dans ce test, la durée d'action du SR 57746 A, après une administration orale de 3 mg/kg, est supérieure à 7 heures.

### Exemple 2

Un principe actif représentatif de la présente invention, le SR 57746A, soumis au test du Conflit Geller-Seifter (C.G-S) chez le rat, selon J. Geller et al. (Psychopharmacologia, Berlin, 1960, I, 482 -492), s'est montré anxiolytique à une dose 10 fois inférieure à la dose sédative. Plus particulièrement, sur les réponses punies, indice d'une activité anxiolytique, le SR 57746 A est actif à une dose de 3 mg/kg per os, alors que sur les réponses non punies, indice d'une activité sédative, il est actif à 30 mg/kg per os, l'activité anorexigène orale chez le rat étant de 10,4 mg/kg.

### Exemple 3

Dans un autre modèle prédictif d'une activité anxiolytique, le test de l'aversion conditionnée à une saveur ("conditioned taste adversion") chez le rat, notamment sur l'aversion induite par le lithium (A.L.) , selon G.N. Evin et al. (Drug Development Research, 1987, 11, 87-95), le SR 57746 A est actif avec une dose minimale active (d.m.a.) de 3 mg/kg per os.

### Exemple 4

Dans un modèle spécifique prédictif d'une activité antidépressive, le test de la résignation apprise ("learned helplessness") selon A.D. Sherman et al. (Pharmacol. Biochem. Behav., 1982, 16, 449-454), le SR 57746 A est actif chez le rat à une dose orale de 0,5 mg/kg/jour pendant 5 jours, ledit effet étant identique à celui d'une dose orale de 32 mg/kg/jour d'imipramine.

### Exemple 5

On prépare des comprimés contenant 2 ou 4 mg, en base libre, de SR 57746 A, en utilisant, en proportions équivalentes, les excipients de l'exemple 11 de EP 101381.

De la même façon, on prépare des comprimés contenant 2,5, 5 et 10 mg de principe actif.

## Revendications

1. Utilisation de trifluorométhylphényltétrahydropyridines de formule : 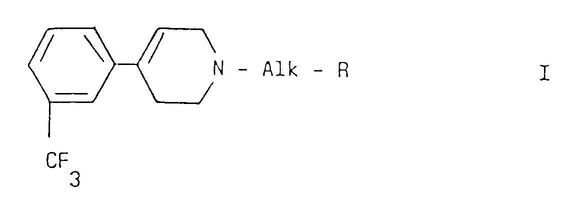 dans laquelle Alk représente un groupe alkylène à chaîne droite ou ramifiée ayant de 1 à 4 atomes de carbone et R représente un groupe cyano, un groupe acétyle, un groupe cycloalkyle ayant 3 à 7 atomes de carbone, un groupe pyridyle, un groupe pyridyle 1-oxyde ou un groupe naphtyle, et de leurs sels pharmaceutiquement acceptables, comme principes actifs pour la préparation de médicaments destinés à combattre l'anxiété et les troubles anxio-dépressifs.

2. Utilisation selon la revendication 1, caractérisée en ce que dans la formule 1 Alk représente un groupe éthylène, propylène ou butylène et R est le groupe cyano.

3. Utilisation selon la revendication 1, caractérisée en ce que dans la formule I Alk est le groupe éthylène et R est un groupe 2-pyridyle, 4-pyridyle, 1-oxyde-4-pyridyle, cyclohexyle ou 2-naphtyle.

4. Utilisation selon la revendication 3, caractérisée en ce que dans la formule 1,R est un groupe 2-naphtyle.

5. Utilisation selon la revendication 4, caractérisée en ce que le principe actif est le chlorhydrate de 1-[2(2-naphtyl)éthyl]-4-(3-trifluorométhylphényl)-1,2,3,6-tétrahydropyridine.

## Claims

1. Use of trifluoromethylphenyltetrahydropyridines of the formula 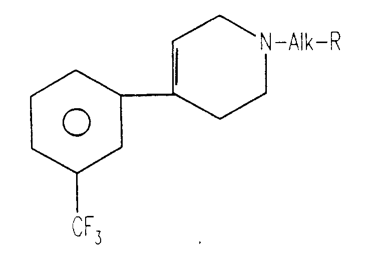 in which Alk represents a straight or branched alkylene group having 1 to 4 carbon atoms and R represents a cyano group, an acetyl group, a cycloalkyl group having 3 to 7 carbon atoms, a pyridyl group, a 1-oxide-pyridyl group or a naphthyl group, and of their pharmaceutically acceptable salts, as active principles for the preparations of drugs intended for the treatment of anxiety and anxio-depressive disorders.

2. Use according to claim 1, characterized in that in formula I Alk represents an ethylene, propylene or buty'lene group and R is the cyano group.

3. Use according to claim 1, characterized in that in formula I Alk is the ethylene group and R is a 2-pyridyl, 4-pyridyl, 1-oxide-4-pyridyl, cyclohexyl or 2-naphthyl group.

4. Use according to claim 3, characterized in that in formula I, R is a 2-naphthyl group.

5. Use according to claim 4, characterized in that the active principle is 1-[2-(2-naphthyl)ethyl]-4-(3-trifluoromethylphenyl)-1,2,3,6-tetrahydropyridine hydrochloride.

## Patentansprüche

1. Verwendung von Trifluormethylphenyltetrahydropyridinen der Formel 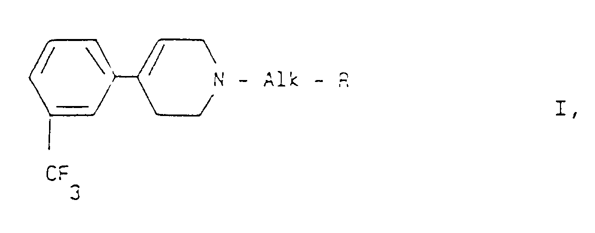 in der bedeuten:
- Alk geradkettiges oder verzweigtes C₁₋₄-Alkylen,
- R Cyano, Acetyl, C₃₋₇-Cycloalkyl, Pyridyl, Pyridyl-1-oxid oder Naphthyl,
sowie ihrer pharmazeutisch akzeptablen Salze als Wirkstoffe für die Herstellung von Arzneimitteln zur Behandlung von Angstgefühlen und depressiven Angstzuständen.

2. Verwendung nach Anspruch 1,
dadurch gekennzeichnet, daß
die Gruppe Alk in Formel I Ethylen, Propylen oder Butylen und die Gruppe R Cyano darstellen.

3. Verwendung nach Anspruch 1,
dadurch gekennzeichnet, daß
die Gruppe Alk in Formel I Ethylen und die Gruppe R 2-Pyridyl, 4-Pyridyl, 4-Pyridyl-1-oxid, Cyclohexyl oder 2-Naphthyl darstellen.

4. Verwendung nach Anspruch 3,
dadurch gekennzeichnet, daß
die Gruppe R in Formel I 2-Naphthyl ist.

5. Verwendung nach Anspruch 4,
dadurch gekennzeichnet, daß
der Wirkstoff das Hydrochlorid von 1-[2-(2-Naphthyl)ethyl]-4-(3-fluormethylphenyl)-1,2,3,6-tetrahydropyridin ist.
